# EUROPEAN PATENT APPLICATION

(11) **EP 4 556 544 A1**
(43) Date of publication of application: **21.05.2025**
(21) Application number: 24195588.9
(22) Date of filing: 21.08.2024
(51) Int. Cl.: C09K 17/42, B01D 53/62

(54) **CARBON CAPTURE SOIL CONDITIONER AND MANUFACTURING METHOD THEREOF**

(30) Priority: 17.11.2023 KR 20230159882; 13.12.2023 KR 20230181052
(71) Applicant: Kyonggi University Industry & Academia Cooperation Foundation, Suwon, Kyeonggi-don 16227 (KR)
(72) Inventor: YANG, Keun Hyeok, 16504 Suwon-si, Gyeonggi-do (KR)
(74) Representative: Isarpatent

(57) **Abstract**

A carbon capture soil conditioner can adsorb carbon dioxide in the atmosphere and also enable soil to be prepared even where soil does not exist previously. The carbon capture soil conditioner includes a porous material to which bacteria with a carbon dioxide adsorption mechanism are adsorbed. The carbon capture soil conditioner further includes a first coating layer that contains natural soil including at least one of clay and red clay fine powder, and a coagulant, and coats a surface of the porous material. The carbon capture soil conditioner further includes a second coating layer that is formed of a coating material containing a silicate-based accelerating agent and coats the surface of the first coating layer. A manufacturing method of the carbon capture soil conditioner is also provided.

## Description

### TECHNICAL FIELD

The disclosure relates to a soil conditioner and a manufacturing method thereof. In particular, the disclosure relates to a carbon capture soil conditioner that contains bacteria with a carbon dioxide adsorption mechanism and adsorbs carbon dioxide in the atmosphere, and to a method for manufacturing the same.

### BACKGROUND

In recent years, as the damage caused by climate change increases, interest in reducing carbon dioxide, which is pointed out as a cause of climate change, is increasing. Accordingly, various efforts are being made to reduce carbon dioxide across various industries. In particular, it is known that cement, which is essential for manufacturing concrete in the construction industry, emits more than 0.8 tons of carbon dioxide per ton produced. Therefore, the construction industry is making efforts to realize carbon neutrality, which reduces the net emission of carbon dioxide to zero as much as it emits carbon dioxide.

In general, soil can store more than twice the amount of carbon dioxide present in the atmosphere and thus play a buffering role in controlling the amount of carbon dioxide in the atmosphere. However, soil suitable for storing carbon dioxide has a lot of sand or gravel so that carbon dioxide can spread quickly, and it is desirable to have a low water table. Unfortunately, there is a limitation in that it is difficult to secure sufficient soil suitable for storing carbon dioxide in nature.

In this regard, Korean Patent No. 10-1313091 discloses a composition for suppressing methane gas emission in soil. According to this, zeolite substituted with alkali ions and alkaline earth metals are used to reduce methane which is one of the greenhouse gases emitted during the process of cultivating rice. However, although this technology can reduce methane generated in the soil, it is difficult to expect a reduction effect of carbon dioxide. In addition, because the methane reduction composition must be sprayed on existing soil, there is a problem that it is difficult to use if sufficient soil is not secured.

### SUMMARY

The disclosure is intended to provide a carbon capture soil conditioner not only capable of adsorbing carbon dioxide in the atmosphere, but also enabling soil to be prepared even where soil does not exist previously. In addition, the disclosure is intended to provide a method for manufacturing the carbon capture soil conditioner.

According to the disclosure, a carbon capture soil conditioner is provided. The carbon capture soil conditioner includes a porous material to which bacteria with a carbon dioxide adsorption mechanism are adsorbed. The carbon capture soil conditioner further includes a first coating layer that contains natural soil including at least one of clay and red clay fine powder, and a coagulant, and coats a surface of the porous material. The carbon capture soil conditioner further includes a second coating layer that is formed of a coating material containing a silicate-based accelerating agent and coats the surface of the first coating layer.

The carbon capture soil conditioner may include the porous material of 5 to 25 wt%, the first coating layer containing the natural soil of 60 to 75 wt% and the coagulant of 5 to 10 wt%, and the second coating layer containing the coating material of 1 to 7 wt%.

The porous material may include at least one of expanded vermiculite and zeolite.

The expanded vermiculite may have a diameter of 1.2 mm or less.

The zeolite may have a diameter of 2.5 to 3.5 µm.

The natural soil may have a particle size of 0.005 to 0.02 mm.

The bacteria may include one or more species selected from the group consisting of Rhodobacter capsulatus, Rhodopseudomonas palustris, Rhodobacter blasticus, and Rhodobacter sphaeroides.

The bacteria may include one or more species selected from the group consisting of Thermoproteus, Sulfolobus, Planctomyces, and Anammox.

The coagulant may include at least one of honey, starch syrup, and grain syrup.

According to the disclosure, a method for manufacturing a carbon capture soil conditioner is provided. The method includes preparing a first coating layer composition by mixing natural soil including at least one of clay and yellow soil fine powder with a coagulant; forming a first coating layer on a surface of a porous material by placing the first coating layer composition into a pill maker together with the porous material on which bacteria having a carbon dioxide adsorption mechanism are adsorbed; and forming a second coating layer by spraying a coating material containing a silicate-based accelerating agent on the surface of the porous material on which the first coating layer is formed.

The soil conditioner according to the disclosure can adsorb carbon dioxide in the atmosphere, using bacteria that adsorb carbon dioxide without being sensitive to anaerobic, aerobic, and light/dark conditions.

The soil conditioner according to the disclosure that uses the porous material to which the bacteria are adsorbed can create an environment in which the bacteria can grow even inside the soil conditioner.

When the soil conditioner according to the disclosure is mixed with natural soil, the coating layers are melted by contact with external moisture and natural soil, and the porous material is exposed to the outside. As a result, the bacteria adsorbed to the porous material can seep into the natural soil, continuously reproduce, and adsorb carbon dioxide in the atmosphere.

### BRIEF DESCRIPTION OF THE DRAWINGS

FIG. 1 is a diagram showing a soil conditioner according to the disclosure.
FIG. 2 is a diagram showing a mechanism in which bacteria adsorb carbon dioxide through the Calvin cycle, according to the disclosure.
FIG. 3 is a diagram showing a mechanism in which bacteria adsorb carbon dioxide through the reverse tricarboxylic acid (TCA) cycle, according to the disclosure.
FIG. 4 is a diagram showing a mechanism in which bacteria adsorb carbon dioxide through the hydroxypropionate cycle, according to the disclosure.
FIG. 5 is a flow chart showing a method for manufacturing a soil conditioner according to the disclosure.
FIG. 6 is a graph showing the carbon dioxide adsorption performance based on the type of bacteria according to experimental examples.
FIG. 7 is a graph showing the carbon dioxide adsorption performance based on the type of porous material according to experimental examples.
FIG. 8 is a graph showing the number of surviving bacteria based on the type of porous material according to experimental examples.
FIG. 9 is a graph showing the carbon dioxide adsorption performance of soil conditioners according to comparative examples and an embodiment.
FIG. 10 is a photograph taken by measuring the number of surviving bacteria of a soil conditioner according to an embodiment.

### DETAILED DESCRIPTION

Hereinafter, various embodiments of the disclosure will be described in detail with reference to the accompanying drawings. The disclosure may, however, be embodied in many different forms and should not be construed as being limited to the embodiments set forth herein. Rather, these embodiments are provided so that the disclosure will be thorough and complete and will fully convey the scope of the disclosure to those skilled in the art.

In the following description of embodiments, techniques that are well known in the art and not directly related to the disclosure are not described. This is to clearly convey the subject matter of the disclosure by omitting an unnecessary explanation. In the disclosure, the same or corresponding elements are denoted by the same reference numerals.

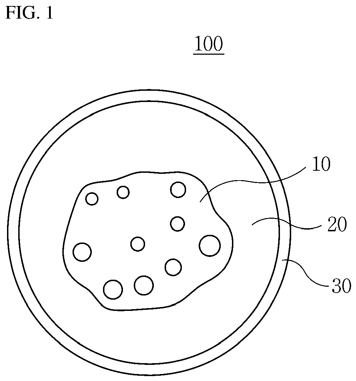

FIG. 1 is a diagram showing a soil conditioner according to the disclosure.

The soil conditioner 100 according to the disclosure includes a porous material 10 to which bacteria with a carbon dioxide adsorption mechanism are adsorbed, a first coating layer 20 that contains natural soil including at least one of clay and red clay fine powder, and a coagulant, and coats the surface of the porous material 10, and a second coating layer 30 that is formed of a coating material containing a silicate-based accelerating agent and coats the surface of the first coating layer 20.

Hereinafter, the above elements of the soil conditioner 100 will be described in more detail.

Bacteria according to the disclosure may have a carbon dioxide adsorption mechanism. In particular, it is preferred that the bacteria according to the disclosure are insensitive to anaerobic, aerobic, and light/dark conditions. The bacteria with these characteristics can be classified into bacteria having a Calvin cycle, a reverse tricarboxylic acid (TCA) cycle, and a hydroxypropionate cycle as carbon dioxide adsorption mechanisms.

First, the bacteria having the Calvin cycle as a carbon dioxide adsorption mechanism may include one or more species selected from the group consisting of Rhodobacter capsulatus, Rhodopseudomonas palustris, Rhodobacter blasticus, and Rhodobacter sphaeroides, which are found in the photosynthetic bacterium Rhodobacter.

Second, the bacteria having the reverse TCA cycle as a carbon dioxide adsorption mechanism may include one or more species selected from the group consisting of Thermoproteus and Sulfolobus.

Third, the bacteria having a hydroxypropionate cycle as a carbon dioxide adsorption mechanism may be a green non-sulfur bacterium, and preferably may contain one or more species selected from the group consisting of Planctomyces and Anammox.

Hereinafter, the carbon dioxide adsorption mechanism of bacteria according to the disclosure will be described in more detail.

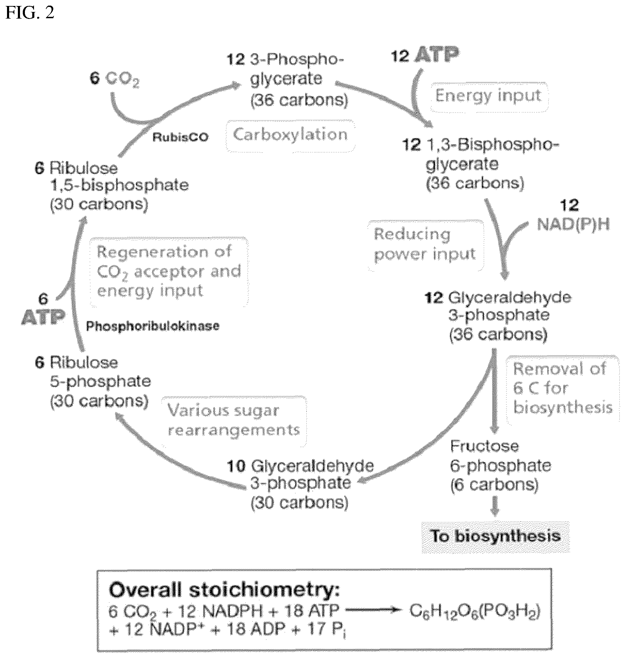

FIG. 2 is a diagram showing a mechanism in which bacteria adsorb carbon dioxide through the Calvin cycle, according to the disclosure.

Referring to FIG. 2, the Calvin cycle adsorbs 6 molecules of carbon dioxide in the process of synthesizing glucose from ATP and NADPH. The Calvin cycle can be roughly divided into three steps. The first step is the fixation of carbon dioxide, catalyzed by an enzyme called rubisco. The rubisco combines ribulose 1,5 diphosphate (RuBP), which consists of 5 carbons with carbon dioxide to form glyceryl triphosphate (3PG) of two molecules. The second step is a process in which glyceryl triphosphate (3PG) is converted to glyceraldehyde triphosphate (G3P) through a reduction process. The glyceraldehyde triphosphate (G3P) thus produced is used to synthesize glucose. Finally, the third step is a reaction in which regeneration of ribulose 1,5 diphosphate (RuBP) occurs. The glyceraldehyde triphosphate (G3P) produced in the second step is converted to ribulose 1,5 diphosphate (RuBP) through ribulose monophosphate (RuMP). The ribulose 1,5 diphosphate (RuBP) thus regenerated may adsorb carbon dioxide while repeating the process of combining with carbon dioxide.

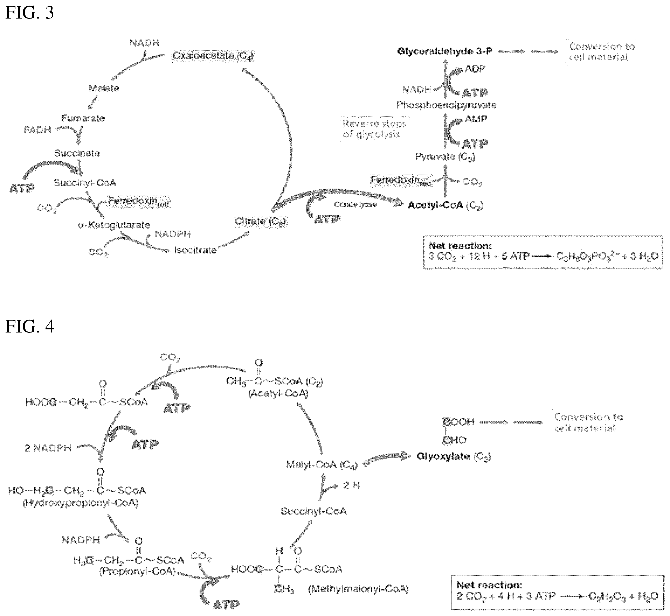

FIG. 3 is a diagram showing a mechanism in which bacteria adsorb carbon dioxide through the reverse TCA cycle, according to the disclosure.

Referring to FIG. 3, the reverse TCA cycle corresponds to turning the TCA cycle in the reverse direction and adsorbs three molecules of carbon dioxide in the process of converting carbon dioxide to acetyl CoA. The reverse TCA cycle uses enzymes such as fumarate reductase, which reduces fumarate to succinate, α-ketoglutarate: ferredoxin oxidoreductase, which transfers electrons received from ferrodoxin to carbon dioxide and succinyl CoA to reduce them to alpha-ketoglutaric acid, and ATP citrate lyase, which breaks down citrate to produce oxaloacetate and acetyl CoA.

FIG. 4 is a diagram showing a mechanism in which bacteria adsorb carbon dioxide through the hydroxypropionate cycle according to the disclosure.

Referring to FIG. 4, the hydroxypropionate cycle adsorbs two molecules of carbon dioxide in the process of converting acetyl CoA to malyl CoA. Referring at the steps of the hydroxypropionate cycle adsorbing carbon dioxide in detail, first, acetyl CoA adsorbs carbon dioxide to form malonyl CoA, and malonyl CoA is converted to propionyl CoA through a hydroxypropionate, which is an intermediate. Then, in the process of carboxylation of propionyl CoA to methylmalonyl CoA, carbon dioxide is adsorbed, and methylmalonyl CoA is isomerized to succinyl CoA. Then, the succinyl CoA forms malyl CoA, and the malyl CoA is decomposed into acetyl CoA and glyoxylate.

A 3-hydroxypropionate/4-hydroxybutyrate cycle and a dicarboxylate/4-hydroxybutyrate cycle are known as a carbon dioxide adsorption mechanism in which the hydroxypropionate cycle is modified.

The soil conditioner 100 according to the disclosure can adsorb carbon dioxide in the atmosphere, using bacteria that adsorb carbon dioxide without being sensitive to anaerobic, aerobic, and light/dark conditions.

If such bacteria are simply added when manufacturing the soil conditioner 100, the bacteria are likely to die due to friction and impact during the mixing process of the soil conditioner 100. Therefore, the soil conditioner 100 according to the disclosure uses the porous material 10 to which the bacteria are adsorbed, so that it is possible to create an environment in which the bacteria can grow even inside the soil conditioner 100.

The porous material 10 may include at least one of expanded vermiculite and zeolite, which have excellent cation exchange capabilities. For example, the porous material 10 may include at least one of expanded vermiculite with a diameter of 1.2 mm or less and zeolite with a diameter of 2.5 to 3.5 µm.

The porous material 10 may preferably have an effective moisture content of 40 vol% or more and a porosity of 50 vol% or more, thereby creating an environment in which the bacteria can grow. In particular, the porous material 10 has the property of adsorbing organic matter due to exchangeable cations (Mg²⁺, Ca²⁺) present on the surface, so it can absorb bacteria and culture media necessary for bacterial growth.

The first coating layer 20 may include natural soil and a coagulant. Here, the natural soil may include at least one of clay and red clay fine powder. If the particle size of the natural soil is less than 0.005 mm, the natural soil may block the pores of the porous material 10, thereby inhibiting the growth of bacteria adsorbed on the porous material 10. If the particle size is more than 0.02 mm, the natural soil may not be coagulated by the coagulant. Therefore, it is desirable for the natural soil to have a particle size of 0.005 to 0.02 mm.

The coagulant can coagulate the natural soil so that the natural soil is coated on the porous material 10. The coagulant may include at least one of honey, starch syrup, and grain syrup. The coagulant not only coagulates and coats the natural soil, but also acts as nutrient for bacteria, thus providing an environment in which the bacteria can initially grow stably inside the soil conditioner 100.

The first coating layer 20 may be coated on the surface of the porous material 10 to form a pill-like shape. However, since the first coating layer 20 can easily fall off from the porous material 10, the pill-like shape of the soil conditioner 100 can easily collapse. Therefore, in order to temporarily maintain the pill-like shape of the soil conditioner 100, the soil conditioner 100 according to the disclosure includes the second coating layer 30 that coats the surface of the first coating layer 20.

The second coating layer 30 may coat the surface of the first coating layer 20 coated on the surface of the porous material 10. The second coating layer 30 may be formed of, but is not limited to, a coating material containing a silicate-based accelerating agent whose main ingredient is sodium silicate hydrate (Na₂SiO₃·nHiO).

The soil conditioner 100 according to the disclosure can adjust the amount of carbon that can be absorbed by the bacteria adsorbed on the porous material 10, depending on the thickness of the first and second coating layers 20 and 30. For example, the soil conditioner 100 according to the disclosure may include the porous material 10 of 5 to 25 wt%, the first coating layer 20 containing the natural soil of 60 to 75 wt% and the coagulant of 5 to 10 wt%, and the second coating layer 30 containing the coating material of 1 to 7 wt%.

The soil conditioner 100 according to the disclosure can be used by being mixed with existing natural soil or used as a substitute for artificial lightweight soil.

In particular, the soil conditioner 100 according to the disclosure is formed in a pill-like shape and can initially maintain its shape due to the second coating layer 30, but the pill-like shape may collapse over time to expose the first coating layer 20 to the outside. Afterwards, as the first coating layer 20 is peeled off, the porous material 10 may be exposed.

Therefore, when the soil conditioner 100 according to the disclosure is mixed with natural soil, the coating layers 20 and 30 are melted by contact with external moisture and natural soil, and the porous material 10 is exposed to the outside. As a result, the bacteria adsorbed to the porous material 10 can seep into the natural soil, continuously reproduce, and adsorb carbon dioxide in the atmosphere.

Hereinafter, the manufacturing method of the soil conditioner according to the disclosure will be described in more detail.

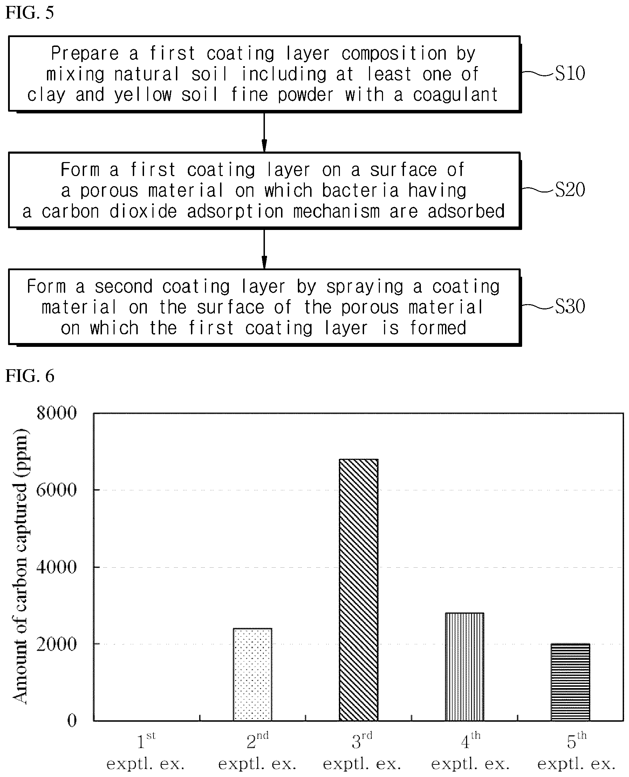

FIG. 5 is a flow chart showing a method for manufacturing a soil conditioner according to the disclosure.

Referring to FIG. 5, in step S10, a first coating layer composition is prepared by mixing natural soil including at least one of clay and yellow soil fine powder with a coagulant.

Here, the natural soil may have a particle size of 0.005 to 0.02 mm, and the coagulant may include at least one of honey, starch syrup, and grain syrup.

Next, in step S20, the first coating layer composition is placed into a pill maker together with a porous material on which bacteria having a carbon dioxide adsorption mechanism are adsorbed, to form a first coating layer on the surface of the porous material.

Here, the porous material may include at least one of expanded vermiculite having a diameter of 1.2 mm or less and zeolite having a diameter of 2.5 to 3.5 µm.

The bacteria may include one or more species selected from the group consisting of Rhodobacter capsulatus, Rhodopseudomonas palustris, Rhodobacter blasticus, and Rhodobacter sphaeroides. Alternatively or additionally, the bacteria may include one or more species selected from the group consisting of Thermoproteus, Sulfolobus, Planctomyces, and Anammox.

The porous material can be sterilized using nanobubble water, completely immersed in a culture medium containing bacteria at a weight ratio of 1:3 to 4, and then subjected to negative pressure so that the bacteria are adsorbed. Here, the process of applying negative pressure may be performed at 10 to 30 torr for 20 to 40 minutes in a negative pressure container, but is not limited thereto.

The nanobubble water used to sterilize the porous material is formed of ultrafine water bubbles each of which contains a large amount of oxygen. Therefore, washing the porous material with the nanobubble water can increase the amount of dissolved oxygen inside the porous material, thereby enhancing the activity of photosynthetic bacteria. In addition, the nanobubble water can remove foreign substances attached to the surface of the porous material, and since hydroxyl radicals generated in water have a (-) property, they can be adsorbed to (+) ions, which are pollutants, and remove pollutants. The nanobubble water forms molecules such as ozone (O₃) and hydroxyl groups (-OH) by activating oxygen in the air while nanobubbles are generated, and these molecules can act as sterilizing agents that destroy organisms and volatile organic compounds. The porous material can be dried at 100°C for 6 to 10 hours after being washed with the nanobubble water, but is not limited thereto.

In order for the soil conditioner composition according to the disclosure to have a desirable carbon dioxide adsorption rate, the bacteria may be cultured in a culture medium at a concentration of 1×10⁹ cells/mL or more in an incubator at 5 to 50°C. Here, the culture medium may include, but is not limited to, yeast extract, disodium succinate hexahydrate, and potassium dihydrogen phosphate (KH₂PO₄). For example, per 1 L of purified water, the culture medium may contain 1.0 g/L yeast extract, 1.0 g/L disodium succinate hexahydrate, 0.5 mL/L ethanol, 1 mL/L ferric citrate solution (0.5%), 0.5 g/L potassium dihydrogen phosphate (KH₂PO₄), 0.4 g/L magnesium sulfate (MgSO₄·7H₂O), 0.4 g/L sodium chloride (NaCl), 0.4 g/L ammonium chloride (NH₄Cl), 0.05 g/L calcium chloride (CaCl₂·2H₂O), and 1 mL/L trace element solution (SL-6).

The porous material on which bacteria are adsorbed can be placed in a rotary pill maker together with the first coating layer composition to form a first coating layer on the surface. At this time, the pill maker may be rotated at a speed of 20 to 40 rpm for 30 seconds to 2 minutes to form the first coating layer on the surface of the porous material.

Finally, in step S30, to form a second coating layer, a coating material containing a silicate-based accelerating agent is sprayed on the surface of the porous material on which the first coating layer is formed.

At this time, the second coating layer can be formed by using a compression sprayer for spraying the coating material on the surface of the porous material on which the first coating layer is formed.

### [Experimental Examples]

### Evaluation of carbon dioxide adsorption performance based on the type of bacteria

In order to evaluate the carbon dioxide adsorption performance based on the type of bacteria according to the disclosure, media according to the first to fifth experimental examples were prepared.

The medium used in the first experimental example is a basic medium in which bacteria are not cultured. In the medium of the second experimental example, Rhodobacter capsulatus were cultured. In the medium of the third experimental example, Rhodopseudomonas palustris were cultured. In the medium of the fourth experimental example, Rhodobacter blasticus were cultured. In the medium of the fifth experimental example, Rhodobacter sphaeroids were cultured.

The medium according to each of the first to fifth experimental examples was placed in a sealed container, and carbon dioxide was added to the container to form a carbon dioxide saturation state. Then, after 24 hours, to measure the amount of carbon dioxide captured by the bacteria cultured in each medium, the amount of carbon dioxide that had decreased compared to the initial amount was measured.

FIG. 6 is a graph showing the carbon dioxide adsorption performance based on the type of bacteria according to experimental examples.

Referring to FIG. 6, it can be seen that no carbon dioxide was captured in the first experimental example, whereas 2,400 ppm of carbon dioxide was captured in the second experimental example, 6,800 ppm of carbon dioxide was captured in the third experimental example, 2,800 ppm of carbon dioxide was captured in the fourth experimental example, and 2,000 ppm of carbon dioxide was captured in the fifth experimental example. In other words, it can be seen that Rhodopseudomonas palustris has the best carbon dioxide adsorption performance.

### Evaluation of carbon dioxide adsorption performance based on the type of porous material

In order to evaluate the carbon dioxide adsorption performance based on the type of porous material according to the disclosure, porous materials according to the 6^{th} to 15^{th} experimental examples were manufactured.

The porous material of the 6^{th} experimental example is expanded vermiculite, the porous material of the 7^{th} experimental example is expanded vermiculite adsorbed with Rhodobacter capsulatus, the porous material of the 8^{th} experimental example is expanded vermiculite adsorbed with Rhodopseudomonas palustris, the porous material of the 9^{th} experimental example is expanded vermiculite adsorbed with Rhodobacter blasticus, and the porous material of the 10^{th} experimental example is expanded vermiculite adsorbed with Rhodobacter spheroides.

In addition, the porous material of the 11^{th} experimental example is a zeolite, the porous material of the 12^{th} experimental example is a zeolite adsorbed with Rhodobacter capsulatus, the porous material of the 13^{th} experimental example is a zeolite adsorbed with Rhodopseudomonas palustris, the porous material of the 14^{th} experimental example is a zeolite adsorbed with Rhodobacter blasticus, and the porous material of the 15^{th} experimental example is a zeolite adsorbed with Rhodobacter spheroides.

The porous material according to each of the 6^{th} to 15^{th} experimental examples was placed in a sealed container, and carbon dioxide was added to the container to form a carbon dioxide saturation state. Then, after 24 hours, to measure the amount of carbon dioxide captured by each porous material, the amount of carbon dioxide that had decreased compared to the initial amount was measured.

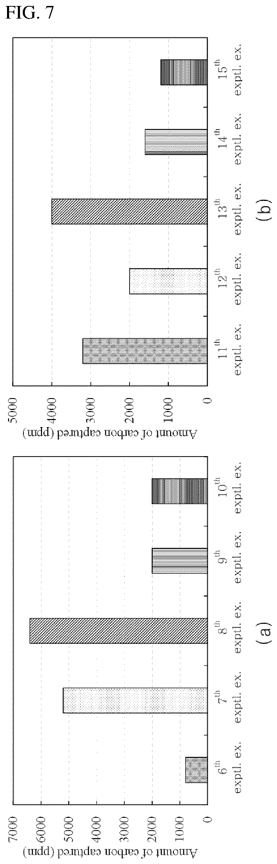

FIG. 7 is a graph showing the carbon dioxide adsorption performance based on the type of porous material according to experimental examples.

Referring to FIG. 7, it can be seen that the porous material of the 6^{th} experimental example captures 800 ppm of carbon dioxide, and the porous material of the 11^{th} experimental example captures 3,200 ppm of carbon dioxide. That is, it can be seen that the carbon dioxide adsorption performance of zeolite is superior to that of expanded vermiculite. However, it can be seen that the porous materials of the 7^{th} to 10^{th} experimental examples capture 2,000 to 6,800 ppm of carbon dioxide, and the porous materials of the 12^{th} to 15^{th} experimental examples capture 1,200 to 4,000 ppm of carbon dioxide. That is, it can be seen that the carbon dioxide adsorption performance of expanded vermiculite adsorbed with bacteria is superior to that of zeolite adsorbed with bacteria. In particular, it can be seen that the carbon dioxide adsorption performance of expanded vermiculite adsorbed with Rhodopseudomonas palustris is the best.

### Evaluation of bacterial growth based on the type of porous material

In order to evaluate the growth of bacteria based on the type of porous material according to the disclosure, the number of surviving bacteria existing in the porous material according to the 6^{th} to 15^{th} experimental examples was measured.

1 g of the porous material according to each of the 6^{th} to 15^{th} experimental examples was collected and placed in 50 ml of inorganic medium, and then bacteria were removed from the porous material in a stirrer at 30°C for 3 hours. Thereafter, 100 mg of a sample in which a culture medium of the removed bacteria was diluted at a ratio of 10⁻⁶ was inoculated onto an agar medium and cultured for 3 days. Based on the results of counting the bacterial colonies formed through re-cultivation on the agar medium, the number of surviving bacteria existing in the porous material according to the 6^{th} to 15^{th} experimental examples was measured.

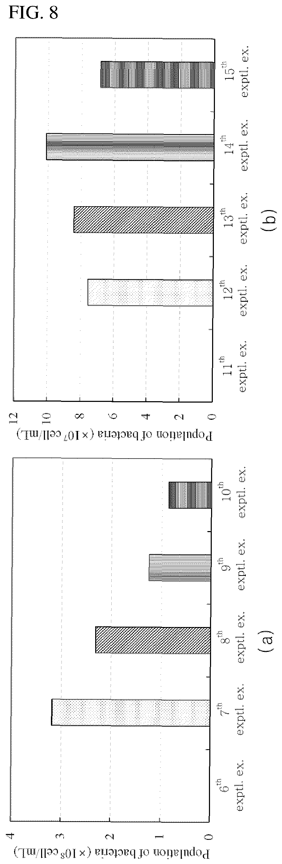

FIG. 8 is a graph showing the number of surviving bacteria based on the type of porous material according to experimental examples.

Referring to FIG. 8, it can be seen that the number of surviving bacteria existing in the porous material according to each of the 7^{th} to 10^{th} experimental examples and the 12^{th} to 15^{th} experimental examples ranges from 7.23 × 10⁷ to 3.18 × 10⁸.

### [Comparative Example and Embodiment]

In order to confirm the characteristics of the soil conditioner according to the disclosure, soil conditioners according to comparative examples and an embodiment were prepared.

A soil conditioner according to the first comparative example was prepared using only clay, and a soil conditioner according to the second comparative example was prepared by coating a first coating layer mixed with clay and honey on the surface of expanded vermiculite. Also, a soil conditioner according to the embodiment was prepared by coating a first coating layer mixed with clay and honey on the surface of expanded vermiculite adsorbed with Rhodopseudomonas palustris.

The porous material according to each of the first and second comparative examples and the embodiment was placed in a sealed container, and carbon dioxide was added to the container to form a carbon dioxide saturation state. Then, after 24 hours, to measure the amount of carbon dioxide captured by each soil conditioner, the amount of carbon dioxide that had decreased compared to the initial amount was measured.

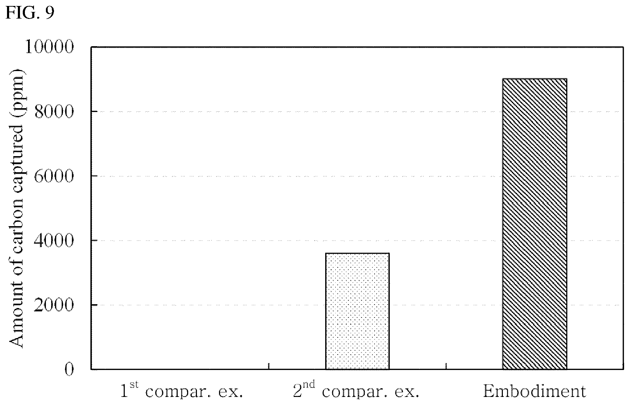

FIG. 9 is a graph showing the carbon dioxide adsorption performance of soil conditioners according to comparative examples and an embodiment.

Referring to FIG. 9, it can be seen that the soil conditioner of the first comparative example did not capture carbon dioxide, and the soil conditioner of the second comparative example captured 3,400 ppm of carbon dioxide. On the other hand, it can be seen that the soil conditioner of the embodiment captured 9,000 ppm of carbon dioxide. It can be seen that the soil conditioner of the embodiment captured 4,800 to 9,000 ppm of carbon dioxide although the amount of carbon dioxide captured varied depending on the thickness of the coating layer. That is, it can be seen that the soil conditioner including the porous material to which bacteria are adsorbed has excellent carbon dioxide adsorption performance.

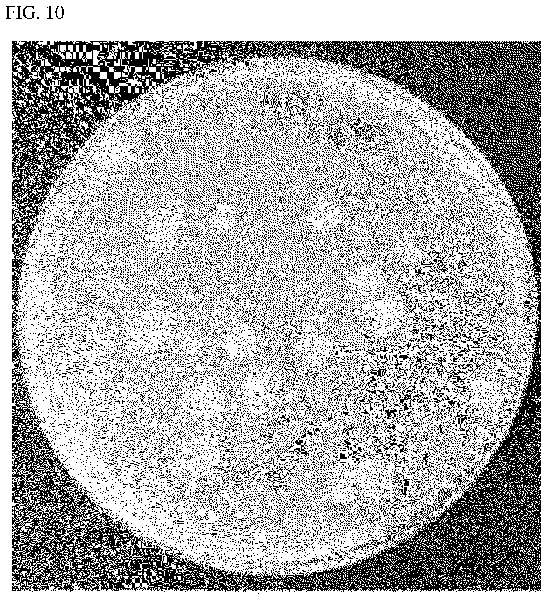

FIG. 10 is a photograph taken by measuring the number of surviving bacteria of a soil conditioner according to an embodiment.

As shown in FIG. 10, the number of surviving bacteria present in the soil conditioner according to the embodiment was measured, and it was seen that 4.5±0.42 × 10⁴ cells/mL of bacteria survived.

While the present invention has been particularly shown and described with reference to exemplary embodiments thereof, it will be understood by those skilled in the art that various changes in form and details may be made therein without departing from the scope of the present invention as defined by the appended claims.

This research was supported by "STEAM Research (R&D) / Future Promising Convergence Technology Pioneer (challenge type)" through the National Research Foundation of Korea (NRF) funded by the Ministry of Science and ICT (Project Number: 1711195861).

This research was also supported by "Industrial Technology Alchemist Project" through the Korea Planning & Evaluation Institute of Industrial Technology (KEIT) funded by the Ministry of Trade, Industry and Energy (Project Number: 1415187362).

This research was also supported by "Support for Activation of Industry-Academia-Research Cooperation (R&D) / Promotion of University Technology Management" through the Commercialization Promotion Agency for R&D Outcomes (COMPA) funded by the Ministry of Science and ICT (Project Number: Not assigned).

## Claims

1. A carbon capture soil conditioner comprising:
a porous material to which bacteria with a carbon dioxide adsorption mechanism are adsorbed;
a first coating layer that contains natural soil including at least one of clay and red clay fine powder, and a coagulant, and coats a surface of the porous material; and
a second coating layer that is formed of a coating material containing a silicate-based accelerating agent and coats the surface of the first coating layer.

2. The carbon capture soil conditioner of claim 1, comprising:
the porous material of 5 to 25 wt%, the first coating layer containing the natural soil of 60 to 75 wt% and the coagulant of 5 to 10 wt%, and the second coating layer containing the coating material of 1 to 7 wt%.

3. The carbon capture soil conditioner of claim 2, wherein the porous material includes at least one of expanded vermiculite and zeolite.

4. The carbon capture soil conditioner of claim 3, wherein the expanded vermiculite has a diameter of 1.2 mm or less.

5. The carbon capture soil conditioner of claim 3, wherein the zeolite has a diameter of 2.5 to 3.5 µm.

6. The carbon capture soil conditioner of claim 2, wherein the natural soil has a particle size of 0.005 to 0.02 mm.

7. The carbon capture soil conditioner of claim 2, wherein the bacteria includes one or more species selected from the group consisting of Rhodobacter capsulatus, Rhodopseudomonas palustris, Rhodobacter blasticus, and Rhodobacter sphaeroides.

8. The carbon capture soil conditioner of claim 2, wherein the bacteria includes one or more species selected from the group consisting of Thermoproteus, Sulfolobus, Planctomyces, and Anammox.

9. The carbon capture soil conditioner of claim 1, wherein the coagulant includes at least one of honey, starch syrup, and grain syrup.

10. A method for manufacturing a carbon capture soil conditioner, the method comprising:
preparing a first coating layer composition by mixing natural soil including at least one of clay and yellow soil fine powder with a coagulant;
forming a first coating layer on a surface of a porous material by placing the first coating layer composition into a pill maker together with the porous material on which bacteria having a carbon dioxide adsorption mechanism are adsorbed; and
forming a second coating layer by spraying a coating material containing a silicate-based accelerating agent on the surface of the porous material on which the first coating layer is formed.
